# EUROPEAN PATENT SPECIFICATION

(11) **EP 4 070 748 B1**
(45) Date of publication and mention of the grant of the patent: **10.07.2024**
(21) Application number: 22166902.1
(22) Date of filing: 06.04.2022
(51) Int. Cl.: A61B 17/86

(54) **SPINAL SCREW**
WIRBELSÄULENSCHRAUBE
VIS RACHIDIENNE

(30) Priority: 09.04.2021 US 202117226171
(43) Date of publication of application: 12.10.2022
(73) Proprietor: Globus Medical, Inc, Audubon, PA 19403 (US)
(72) Inventor: LAMERS, Stefan P., Phoenixville, PA 19460 (US); BECHTEL, Matthew, Philadelphia, PA 19147 (US); PAUL, Jeshurun S., Phoenixville, PA 19460 (US); PAUL, David C., Phoenixville, PA 19460 (US)
(74) Representative: Morabito, Sara

(56) References cited:
- CA-A1- 3 098 391
- US-A1- 2011 004 256
- US-A1- 2018 146 987
- US-A1- 2019 269 469
- US-A1- 2019 374 269

## Description

### FIELD OF THE INVENTION

The present invention relates to a system for stabilizing the spine, and more particularly to a spinal screw that reduces the risk of bone fracturing and reduces spinal screw insertion torque.

### BACKGROUND OF THE INVENTION

The human spine consists of individual vertebras that are connected to each other. Under normal circumstances, the structures that make up the spine function to protect the neural structures and to allow us to stand erect, bear axial loads, and be flexible for bending and rotation. However, disorders of the spine occur when one or more of these spine structures are abnormal. In these pathologic circumstances, surgery may be tried to restore the spine to normal, achieve stability, protect the neural structures, or to relieve the patient of discomfort. The goal of spine surgery for a multitude of spinal disorders, especially those causing compression of the neural structures, is often decompression of the neural elements and/or fusion of adjacent vertebral segments. Fusion works well because it stops pain due to movement at the facet joints or intervertebral discs, holds the spine in place after correcting deformity, and prevents instability and or deformity of the spine after surgical procedures such as discectomies, laminectomies, or corpectomies.

Several spinal fixation systems exist for stabilizing the spine so that bony fusion is achieved. The majority of these fixation systems utilize fixation elements such as rods, wires, or plates that attach to screws threaded into the vertebral bodies, facets, or the pedicles. Because the outer surface of the vertebral body is typically non-planar and the structure of the vertebras is relatively complex, it is important that the fixation elements (e.g., rods, plates, wires, staples and/or screws) are properly aligned when they are inserted into the vertebras. Improper alignment may result in improper or unstable placement of the fixation element and/or disengagement of the fixation element.

Achieving and maintaining accurate positioning and guidance of these fixation elements, however have proven to be quite difficult in practice. Such positioning difficulties are further complicated by the fact that the alignment angle for a fixation device through one vertebral body or pair of vertebral bodies will be unique to that individual due to individual differences in the spinal curvature and anatomies. Accordingly, there is a need for a spinal screw assembly that reduces insertion torque of a self-drilling screw design without compromising fixation attributes. Thus, there is a need for a self-drilling spinal screw that reduces the risk of bone fracturing during insertion, reduce the need for drilling and tapping, and reduce overall operating room time.

US 2019/374269, CA 3 098 391 and US 2019/269469 all describe spinal screws known in the art.

### SUMMARY OF THE INVENTION

The disclosure meets the foregoing need and uses spinal screws to reduce insertion torque and risk of bone fracturs during the placement of the spinal screws into bone.

According to the invention it is provided a spinal screw according to claim 1. Further advantageous aspects of the invention are set forth in the dependent claims.

Additional features, advantages, and embodiments of the disclosure may be set forth or apparent from consideration of the following detailed description, drawings, and claims. Moreover, it is to be understood that both the foregoing summary of the disclosure and the following detailed description are exemplary and intended to provide further explanation without limiting the scope of the disclosure as claimed.

A spinal screw for positioning within bone for use in surgical procedures, the spinal screw having a head and a shaft, the shaft having a proximal end and a distal end. A through-hole extends from the head through the shaft to the distal end of the shaft. The through-hole defines a longitudinal axis of the spinal screw and a spring is positioned within the shaft. A retractable tip element is also positioned within the through hole of the shaft and is operationally coupled to the spring.

### BRIEF DESCRIPTION OF THE DRAWINGS

A more complete understanding of the present invention, and the attendant advantages and features thereof, will be more readily understood by reference to the following detailed description when considered in conjunction with the accompanying drawings, wherein:
FIG. 1 is perspective view of a spinal screw in accordance with exemplary embodiments of the present disclosure;
FIG. 2 illustrates the spinal screw of FIG. 1 in accordance with exemplary embodiments of the present disclosure .
FIG. 3 is a perspective view of a spinal screw;
FIGS. 4 and 5 are views of the distal end of a spinal screw;
FIGS. 6 and 7 are view of the distal end of a spinal screw in accordance with exemplary embodiments of the present disclosure;
FIGS. 8 and 9 illustrate yet another example of a spinal screw;
FIGS. 10 and 11 illustrate a spinal screw in accordance with exemplary embodiments of the present disclosure; and

### DETAILED DESCRIPTION OF THE INVENTION

It is to be understood that the present disclosure is not limited in its application to the details of construction and the arrangement of components set forth in the description herein or illustrated in the drawings. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless specified or limited otherwise, the terms "mounted," "connected," "supported," and "coupled" and variations thereof are used broadly and encompass both direct and indirect mountings, connections, supports, and couplings. Further, "connected" and "coupled" are not restricted to physical or mechanical connections or couplings.

The following discussion is presented to enable a person skilled in the art to make and use embodiments of the present disclosure. The following detailed description is to be read with reference to the figures, in which like elements in different figures have like reference numerals. The figures, which are not necessarily to scale, depict selected embodiments and are not intended to limit the scope of the embodiments.

Additional aspects, advantages and/or other features of example embodiments of the invention will become apparent in view of the following detailed description. It should be apparent to those skilled in the art that the described embodiments provided herein are merely exemplary and illustrative and not limiting.

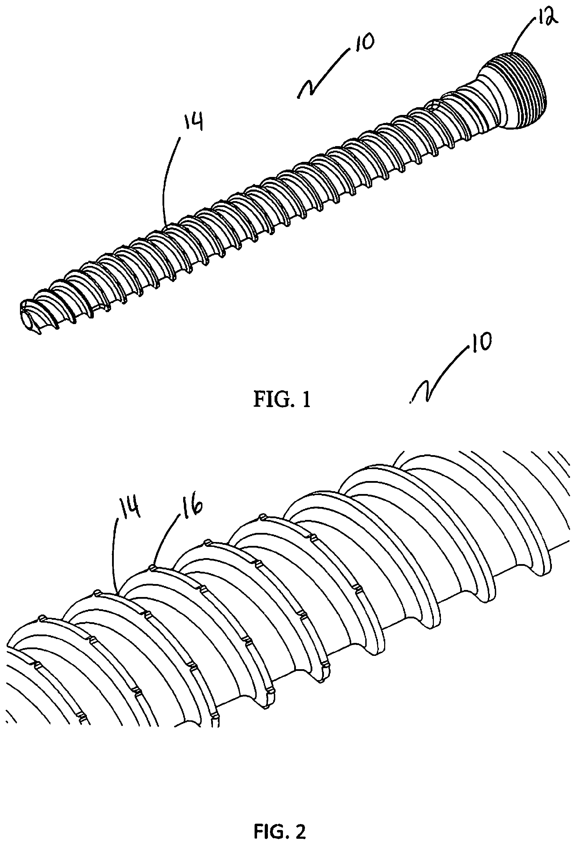

FIGS. 1 and 2 illustrate a spinal screw 10 configured to be used in surgical procedures. The spinal screw 20 comprises a head portion 12 and shaft portion 14. The shaft portion 14 is provided with threading to enable the spinal screw 10 to be rotated into bone. The shaft portion 14 in this particular embodiment is provided with protrusions 16 extending from the threads of the shaft portion 14, as more clearly shown in FIG. 2. The protrusions 16 provide an external cutting peak that is slightly over the major diameter of the spinal screw 10. The protrusions 16 allow for the decrease in the insertion torque of the spinal screw 10 while effectively eliminating the need to drill and tap within the bone prior to the spinal screw 10 insertion. The spinal screw 10 as illustrated is provided with a self-tapping thread profile and a pitch that enables the spinal screw 10 to be a self-drilling and tapping screw. It should be noted the spinal screw 10 may be configured in various lengths, and diameters. Also, the protrusions 16 may be modified in various shapes such as hooks, pyramids, rounded edges, and any configuration that allows for the decrease in the insertion torque of the spinal screw 10.

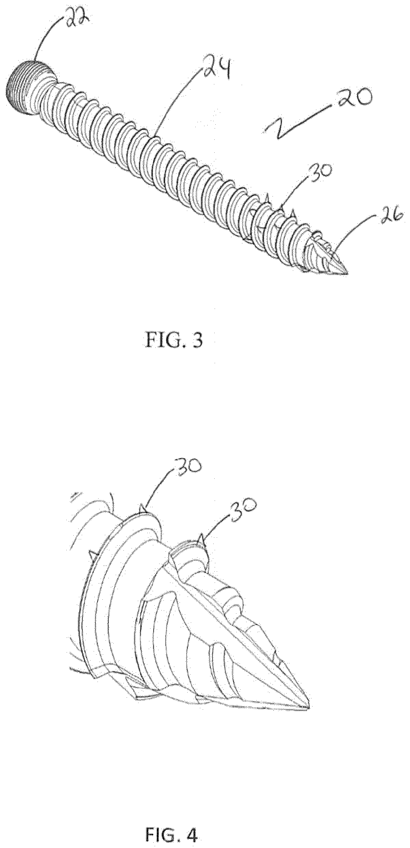

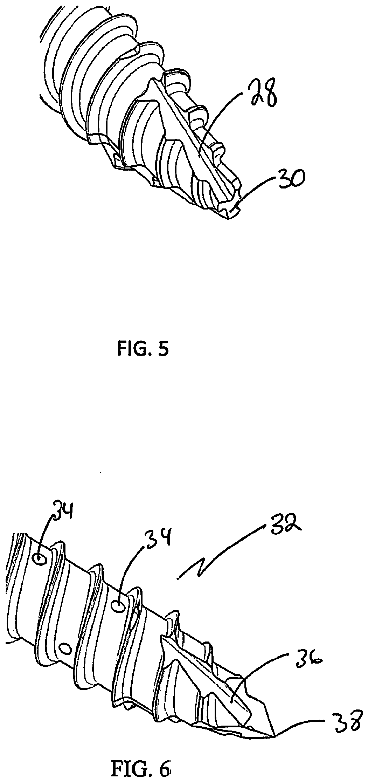

FIG. 3 illustrates yet another spinal screw 20 with a head portion 22 and a shaft portion 24. The distal end of the shaft portion 24 is configured with a cutting flute 26 and configured to cut bone. FIG. 4 illustrates a closer view of the cutting flute 26 in accordance with an exemplary embodiment of the present invention. The distal end of the shaft portion 24 in this embodiment provides a cutting flute 26 with a sharp tip. In another embodiment the distal end of the shaft portion 24 of a spinal screw is provided with a cutting flute 28 with blunt flat end 30 as shown in FIG. 5. The cutting flutes shown in FIG. 4 and 5 are configured to decrease the insertion torque of the spinal screw while effectively eliminating the need to drill and tap prior to the spinal screw insertion into bone. The spinal screws of FIG. 4 and 5 may also be provided with protrusions 32 to decrease the insertion torque required for insertion of the spinal screw into bone. These protrusions may be configured to extend the entire length of the shaft portion of the spinal screw or localized to different sections of the shaft portion of the spinal screw.

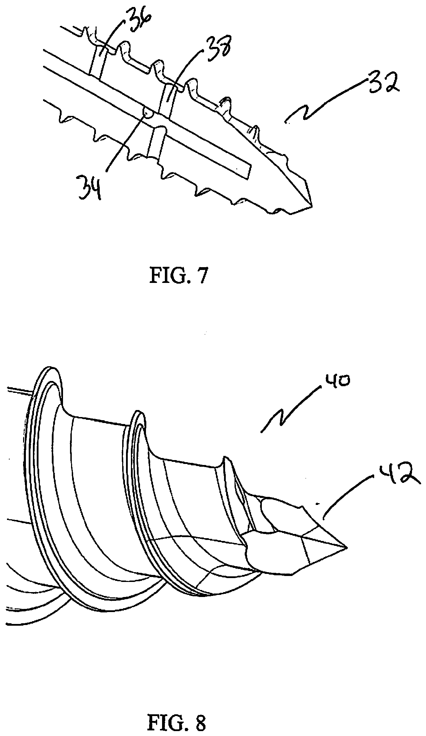

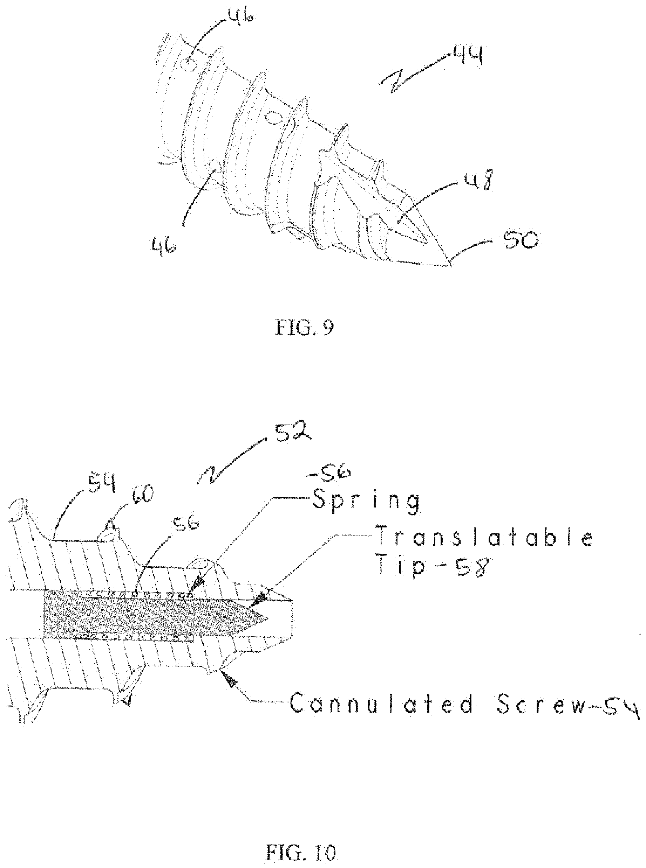

In another embodiment, as shown in FIG. 6. a spinal screw 32 is shown having fenestrations 34, a cutting flute 36, and a sharp tip 38. FIG. 7 illustrates a cross sectional view of the spinal screw 32 illustrating the fenestrations 34. The fenestrations as shown can be configured as blind holes 36 or through holes 38 extending through the shaft of the spinal screw 32. In another example, FIG. 8 illustrates a spinal screw 40 that includes a threaded trocar tip 42 and FIG. 9 illustrates the spinal screw 44 with fenestrations 46, a cutting flute 48 and the threaded trocar tip 50. It should be noted that the spinal screw shown in all the above examples may also be cannulated. The fenestration and the cannulations provided in the screw enable the insertion of bone cement, autograft, allograft or any combination thereof.

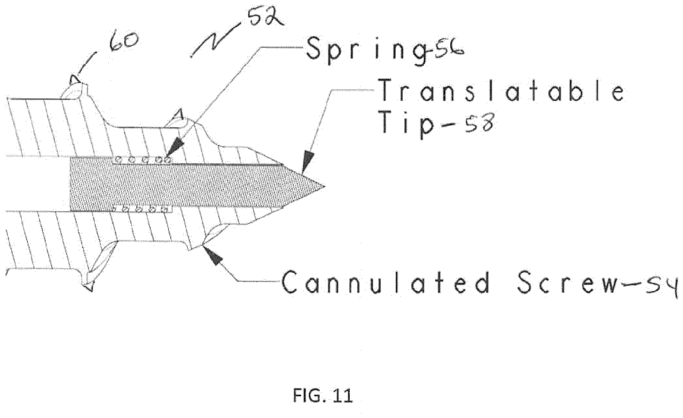

Now, turning to FIGS. 10 and 11, a multi-piece spinal screw 52 is provided . Spinal screw 52 includes a cannulated screw portion 54, an internal spring 56, and internal translatable tip 58 centered withing the cannulated screw 54. The cannulated screw 54 is provide with a head portion and a shaft portion, the shaft portion may be configured with or without a cutting flute. The cannulated screw 54 may also be configured with or without protrusions 60. The internal spring 56 is positioned within a groove on an inner shelf at the distal end of the cannulated screw 54. The translatable tip 58 is positioned within the cannulation of the screw and configured to protrude out the distal end of the cannulated screw as the driver is attached and pushes on the back end of the translatable tip 58. The translatable tip 58 will retract into the cannulated screw 54 after the back of the tip is disengaged. The multi-piece spinal screw 52 as shown in FIGS. 10 and 11 improves the efficiency and the speed at which the bone screw is inserted during surgery. The exemplary embodiment of the spinal screw 52 allows for the better placement of the screw within bone, reduces surgical time, provides a reduction in x-ray exposure, and a reduction in anesthesia exposure over traditional bone screws. The spinal screw according to the present disclosure also reduces the force exerted on the bone reducing the potential cracks and necrosis of the bone.

According to the solution the retractable tip is movable between a first position, when pressure is applied to the instrument, in which the tip of the retractable tip element past the distal end of the shaft to engage with bone and a second position, when pressure on the instrument is no longer applied, in which the retractable tip element is disengaged from the bone and is retracted into the screw. The spring allows for moving the retractable tip into the second position in which the retractable tip is retracted into the screw.

Preferably the spring retracts the tip after the instrument is removed from the spinal screw and no pressure is applied.

In an alternative example, the multipiece retractable tip screw may utilize alternative means for extending and retracting the tip. For example, a ratchet type mechanism may be utilized to extend the tip into bone and retracted by release of the ratchet mechanism. In another embodiments, the tip may be extended various lengths depending on the need of the patient.

The spinal screw as provide in FIGS. 1-11 are configured to be utilized in robotic applications. The spinal screw is configured to be trackable by a robotic system that includes a camera system, and a robotic arm coupled to a robot base. The robotic system includes a computer system that is configured to navigate the spinal screw using trackable markers that are visible to the camera system. The navigation system used by the robotic system also is capable of registering images received by an imaging device such a CT, MRI or fluoroscope to the images received by the camera system. The spinal screw is also configured to attach to trackable markers mechanically and electrically. In another embodiment the spinal screw is provided with bone sensors to determine the density of the bone. In some embodiments, sensors may be positioned on the spine screw to determine bone density and other bone characteristics. In one embodiment, as the spinal screw is threaded into bone, the retractable tip element is retracted into the spinal screw if the sensor no longer senses bone or a reduced bone density.

One skilled in the art will appreciate that the embodiments discussed above are non-limiting. While devices may be described as suitable for a particular location (e.g., vertebra) or approach, one skilled in the art will appreciate that the devices, instruments described herein can be used for multiple locations and approaches. In addition to the devices, instruments described above, one skilled in the art will appreciate that these described features can be used with a number of other implants and instruments, including fixation plates, rods, fasteners, and other orthopedic devices. It will also be appreciated that one or more features of one embodiment may be partially or fully incorporated into one or more other embodiments described herein.

## Claims

1. A spinal screw (20; 52; 32) for positioning within bone for use in surgical procedures, the spinal screw (20; 52; 32) comprising:
- a head (12; 22) and a shaft (14; 24), the shaft (14; 24) having a proximal end and a distal end;
- a through-hole (38) extending from the head (12; 22) through the shaft (14; 24) to the distal end of the shaft (14; 24); the through-hole (38) defining a longitudinal axis of the spinal screw (20; 52; 32);
- a spring (56) positioned within the shaft (14; 24); and
- a retractable tip element (58) positioned within the through hole (38) of the shaft (14; 24) and operationally coupled to the spring (56);
- wherein the retractable tip element (58) includes a first end and a second end, the first end capable of receiving an instrument engaging with the retractable tip (58), the second end having a tip that engages with bone,
- the retractable tip (58) being movable between a first position, when pressure is applied to the instrument, in which the tip of the retractable tip element (58) past the distal end of the shaft (14; 24) to engage with bone and a second position when pressure on the instrument is no longer applied, in which the retractable tip element (58) is disengaged from the bone and is retracted into the screw, wherein the spring is configured to retract the retractable tip (58) after the instrument is removed from the spinal screw and no pressure is applied.

2. The spinal screw (20; 52; 32) of claim 1 wherein the spring (56) is configured to retract the retractable tip (58) into the through hole (38) of the shaft (14; 24)

3. The spinal screw (20; 52; 32) of claim 1, wherein the shaft (14; 24) of the spinal screw (20; 52; 32) includes threads extending from along the length of the shaft (14; 24).

4. The spinal screw (20; 52; 32) of claim 1 or 2, wherein a plurality of protrusions (16; 60) are provided on a major diameter of the spinal screw (20; 52; 32).

5. The spinal screw (20; 52; 32) of any one of the preceding claims, wherein the shaft (14; 24) includes a plurality of fenestrations.

6. The spinal screw (20; 52; 32) of claim 4, wherein the fenestrations include blind holes.

7. The spinal screw (20; 52; 32) of claim 4, wherein the fenestrations include through hole (38) (36)s.

8. The spinal screw (20; 52; 32) of claim 1, wherein the distal tip of the retractable tip element (58) comprises a bone sensor positioned on the distal tip of the retractable tip element (58) and configured to sense a pressure applied on the retractable tip so that the retractable tip element (58) could be retracted when no pressure on the retractable tip is sensed by the bone sensor.

9. The spinal screw (20; 52; 32) of any one of the preceding claims, wherein the spinal screw (20; 52; 32) includes a plurality of tracking markers for use for with a robotic surgical system.

10. The spinal screw (20; 52; 32) of any one of the preceding claims wherein when the tip element protrudes out the distal end of the screw, the edges of the tip of the tip element is continuous with the edge of the screw.

11. The spinal screw (20; 52; 32) of claim 4, wherein the protrusions (16; 60) are configured to reduce insertion torque.

## Patentansprüche

1. Wirbelsäulenschraube (20; 52; 32) zur Positionierung innerhalb eines Knochens zur Verwendung in chirurgischen Verfahren, wobei die Wirbelsäulenschraube (20; 52; 32) aufweist:
- einen Kopf (12; 22) und einen Schaft (14; 24), wobei der Schaft (14; 24) ein proximales Ende und ein distales Ende aufweist;
- ein Durchgangsloch (38), das sich vom Kopf (12; 22) durch den Schaft (14; 24) zum distalen Ende des Schafts (14; 24) erstreckt; wobei das Durchgangsloch (38) eine Längsachse der Wirbelsäulenschraube (20; 52; 32) bildet;
- eine Feder (56), die innerhalb des Schafts (14; 24) angeordnet ist; und
- ein zurückziehbares Spitzenelement (58), das innerhalb des Durchgangslochs (38) des Schafts (14; 24) positioniert und mit der Feder (56) wirkverbunden ist;
- wobei das zurückziehbare Spitzenelement (58) ein erstes Ende und ein zweites Ende umfasst, wobei das erste Ende in der Lage ist, ein Instrument aufzunehmen, das mit der zurückziehbaren Spitze (58) in Eingriff steht, wobei das zweite Ende eine Spitze aufweist, die mit einem Knochen in Eingriff steht,
- wobei die zurückziehbare Spitze (58) zwischen einer ersten Position, wenn Druck auf das Instrument aufgebracht wird, in der die Spitze des zurückziehbaren Spitzenelements (58) über das distale Ende des Schafts (14; 24) hinausgeht, um mit dem Knochen in Eingriff zu kommen, und einer zweiten Position beweglich ist, wenn kein Druck mehr auf das Instrument aufgebracht wird, in der das zurückziehbare Spitzenelement (58) vom Knochen gelöst und in die Schraube zurückgezogen ist, wobei die Feder eingerichtet ist, um die zurückziehbare Spitze (58) zurückzuziehen, nachdem das Instrument von der Wirbelsäulenschraube entfernt wurde und kein Druck mehr aufgebracht wird.

2. Wirbelsäulenschraube (20; 52; 32) nach Anspruch 1, wobei die Feder (56) eingerichtet ist, um die zurückziehbare Spitze (58) in das Durchgangsloch (38) des Schafts (14; 24) zurückzuziehen.

3. Wirbelsäulenschraube (20; 52; 32) nach Anspruch 1, wobei der Schaft (14; 24) der Wirbelsäulenschraube (20; 52; 32) Gewindegänge umfasst, die sich entlang der Länge des Schafts (14; 24) erstrecken.

4. Wirbelsäulenschraube (20; 52; 32) nach Anspruch 1 oder 2, wobei eine Mehrzahl von Vorsprüngen (16; 60) an einem Hauptdurchmesser der Wirbelsäulenschraube (20; 52; 32) vorgesehen ist.

5. Wirbelsäulenschraube (20; 52; 32) nach einem der vorhergehenden Ansprüche, wobei der Schaft (14; 24) eine Mehrzahl von Fensteranordnungen aufweist.

6. Wirbelsäulenschraube (20; 52; 32) nach Anspruch 4, wobei die Fensteranordnungen Sacklöcher umfassen.

7. Wirbelsäulenschraube (20; 52; 32) nach Anspruch 4, wobei die Fensteranordnungen Durchgangslöcher (38) (36) umfassen.

8. Wirbelsäulenschraube (20; 52; 32) nach Anspruch 1, wobei die distale Spitze des zurückziehbaren Spitzenelements (58) einen Knochensensor aufweist, der an der distalen Spitze des zurückziehbaren Spitzenelements (58) positioniert ist, und eingerichtet ist, um einen auf die zurückziehbare Spitze aufgebrachten Druck zu erfassen, sodass das zurückziehbare Spitzenelement (58) zurückgezogen werden kann, wenn kein Druck auf die zurückziehbare Spitze durch den Knochensensor erfasst wird.

9. Wirbelsäulenschraube (20; 52; 32) nach einem der vorhergehenden Ansprüche, wobei die Wirbelsäulenschraube (20; 52; 32) eine Mehrzahl von Verfolgungsmarkierungen zur Verwendung mit einem chirurgischen Robotersystem umfasst.

10. Wirbelsäulenschraube (20; 52; 32) nach einem der vorhergehenden Ansprüche, wobei, wenn das Spitzenelement aus dem distalen Ende der Schraube herausragt, die Kanten der Spitze des Spitzenelements durchgehend mit der Kante der Schraube sind.

11. Wirbelsäulenschraube (20; 52; 32) nach Anspruch 4, wobei die Vorsprünge (16; 60) eingerichtet sind, um das Eindrehmoment zu reduzieren.

## Revendications

1. Vis vertébrale (20 ; 52 ; 32) destinée à un positionnement à l'intérieur d'un os pour une utilisation lors d'opérations chirurgicales, la vis vertébrale (20 ; 52 ; 32) comprenant :
- une tête (12 ; 22) et une tige (14 ; 24), la tige (14 ; 24) comportant une extrémité proximale et une extrémité distale ;
- un trou traversant (38) s'étendant de la tête (12 ; 22) à travers la tige (14 ; 24) jusqu'à l'extrémité distale de la tige (14 ; 24) ; le trou traversant (38) définissant un axe longitudinal de la vis vertébrale (20 ; 52 ; 32) ;
- un ressort (56) positionné à l'intérieur de la tige (14 ; 24) ; et
- un élément pointe rétractable (58) positionné à l'intérieur du trou traversant (38) de la tige (14 ; 24) et accouplé fonctionnellement au ressort (56) ;
- dans laquelle l'élément pointe rétractable (58) comprend une première extrémité et une seconde extrémité, la première extrémité pouvant recevoir un instrument coopérant avec la pointe rétractable (58), la seconde extrémité comportant une pointe qui coopère avec un os,
- la pointe rétractable (58) étant mobile entre une première position, lorsqu'une pression est appliquée à l'instrument, dans laquelle la pointe de l'élément pointe rétractable (58) dépasse l'extrémité distale de la tige (14 ; 24) pour coopérer avec un os et une seconde position, lorsqu'une pression n'est plus appliquée à l'instrument, dans laquelle l'élément pointe rétractable (58) est dégagé de l'os et est rétracté dans la vis, dans laquelle le ressort est configuré pour rétracter la pointe rétractable (58) après le retrait de l'instrument de la vis vertébrale et en l'absence d'une application de pression.

2. Vis vertébrale (20 ; 52 ; 32) selon la revendication 1, dans laquelle le ressort (56) est configuré pour rétracter la pointe rétractable (58) dans le trou traversant (38) de la tige (14 ; 24).

3. Vis vertébrale (20 ; 52 ; 32) selon la revendication 1, dans laquelle la tige (14 ; 24) de la vis vertébrale (20 ; 52 ; 32) comprend des filets s'étendant sur la longueur de la tige (14 ; 24).

4. Vis vertébrale (20 ; 52 ; 32) selon la revendication 1 ou la revendication 2, dans laquelle les saillies d'une pluralité de saillies (16 ; 60) sont ménagées sur un diamètre de la vis vertébrale (20 ; 52 ; 32).

5. Vis vertébrale (20 ; 52 ; 32) selon l'une quelconque des revendications précédentes, dans laquelle la tige (14 ; 24) comprend une pluralité de perforations.

6. Vis vertébrale (20 ; 52 ; 32) selon la revendication 4, dans laquelle les perforations comprennent des trous borgnes.

7. Vis vertébrale (20 ; 52 ; 32) selon la revendication 4, dans laquelle les perforations comprennent un trou traversant (38) (36)s.

8. Vis vertébrale (20 ; 52 ; 32) selon la revendication 1, dans laquelle la pointe distale de l'élément pointe rétractable (58) comprend un capteur d'os positionné sur la pointe distale de l'élément pointe rétractable (58) et configuré pour détecter une pression appliquée à la pointe rétractable de façon à pouvoir rétracter l'élément pointe rétractable (58) lors de la détection, par le capteur d'os, de l'absence de pression appliquée à la pointe rétractable.

9. Vis vertébrale (20 ; 52 ; 32) selon l'une quelconque des revendications précédentes, dans laquelle la vis vertébrale (20 ; 52 ; 32) comprend une pluralité de marques de suivi destinées à être utilisées avec un système chirurgical robotisé.

10. Vis vertébrale (20 ; 52 ; 32) selon l'une quelconque des revendications précédentes, dans laquelle, lorsque l'élément pointe fait saillie hors de l'extrémité distale de la vis, le bord de la pointe de l'élément pointe est contigu au bord de la vis.

11. Vis vertébrale (20 ; 52 ; 32) selon la revendication 4, dans laquelle les saillies (16 ; 60) sont configurées pour réduire un couple d'insertion.
